# EUROPÄISCHE PATENTSCHRIFT

(11) **EP 1 455 810 B1**
(45) Veröffentlichungstag und Bekanntmachung des Hinweises auf die Patenterteilung: **13.06.2007**
(21) Anmeldenummer: 01991845.7
(22) Anmeldetag: 14.12.2001
(51) Int. Cl.: A61K 38/13, A61K 9/107, A61P 1/00, A61P 11/00, A61P 13/00, A61P 17/00, A61P 27/02

(54) **ARZNEIFORMULIERUNG ENTHALTEND CICLOSPORIN UND DEREN VERWENDUNG**
PHARMACEUTICAL FORMULATION COMPRISING CYCLOSPORIN AND USE THEREOF
FORMULATION PHARMACEUTIQUE CONTENANT DE LA CICLOSPORINE ET SON UTILISATION

(43) Veröffentlichungstag der Anmeldung: 15.09.2004
(73) Patentinhaber: JAGOTEC AG, 4132 Muttenz (CH)
(72) Erfinder: WOHLRAB, Johannes, 06120 Halle/S. (DE); NEUBERT, Reinhard, 06120 Halle/S. (DE); JAHN, Konstanze, 39171 Beyendorf (DE)
(74) Vertreter: Pfenning, Meinig & Partner GbR
(86) Internationale Anmeldenummer: PCT/EP2001/014749
(87) Internationale Veröffentlichungsnummer: WO 2003/051385

(56) Entgegenhaltungen:
- WO-A-94/08603
- WO-A-94/08605
- WO-A-99/39700
- DE-A- 10 029 404

## Beschreibung

Die Erfindung betrifft kolloidale Arzneistoffträgersysteme, enthaltend Ciclosporin, zur topischen Applikation auf Haut und Schleimhaut, bestehend aus einer Tensid/Cotensid-Mischung (Polyoxyethylenglycerolmonooleat/Poloxamere), einer hydrophilen Phase, z.B. Propylenglykol/Wasser-Mischungen, einer lipophilen Phase (Isopropylpalmitat bzw. Ölsäure) sowie Penetrationsenhancern.

Diese Erfindung betrifft insbesondere Ciclosporin A (CsA) enthaltende pharmazeutische Zubereitungen zur topischen Applikation für die Therapie krankhafter Veränderungen der Haut, Hautanhangsgebilde oder der Schleimhäute, insbesondere der atopischen Dermatitis und der Psoriasis vulgaris, die Verwendung dieser Zubereitungen und Verfahren zu deren Herstellung.

Es ist bekannt, daß CsA ein aus 11 Aminosäuren bestehendes cyklisches Peptid mit einer molaren Masse von 1202 g/mol ist, das vom Bodenpilz Tolypocladium inflatum produziert wird. CsA ist nur sehr schwer in Wasser (<0,04 mg/ml bei 25°C), dagegen gut in Ölen und Alkoholen löslich. Seine immunmodulierende Wirkung basiert auf der Hemmung der Freisetzung von Interleukin-1 aus Makrophagen und von Interleukin-2 aus T-Helferzellen, die wiederum zytotoxische T-Vorläuferzellen aktivieren, aus denen die zytotoxischen T-Zellen entstehen. Hierbei wird die Transkription der Gene gehemmt, die die genannten Zytokine kodieren. Dabei beeinflußt CsA nur in geringem Maße die körpereigene Abwehr. Aus galenischer Sicht ist der Arzneistoff eine insbesondere für die topische Therapie ungeeignete Substanz, weil die hohe Lipophilie eine Penetration durch die epidermale Lipidbarriere nahezu unmöglich erscheinen läßt. Als Grund für das Scheitern bisheriger Versuche der Herstellung und Anwendung von verschiedenen lipophilen, hydrophilen und liposomalen Präparationen zur topischen Anwendung wird meist die ungenügende Penetration des Arzneistoffs angegeben.

In der Dermatologie hat sich CsA in der systemischen Applikation besonders zur Behandlung der schweren Psoriasis und atopischen Dermatitis bewährt. Darüber hinaus liegen Berichte und Studien über die Wirksamkeit nach systemischer Applikation bei einer Vielzahl weiterer entzündlicher Dermatosen (z.B. Dermatitis ulcerosa, Lichen ruber, aktinisches Retikuloid, disseminiertes Granuloma anulare) vor.

In WO 9302664 werden W/O-Mikroemulsionen beschrieben, die eine lipophile Phase (mittelkettige Triglyceride und ein Tensid mit niedrigem HLB-Wert im Verhältnis 5:1 bis 1,5:1), eine wässrige hydrophile Phase, ein Tensid mit hohem HLB-Wert sowie ein wasserlösliches therapeutisches Agens enthalten.

Die GB 2222770 umfasst Mikroemulsions-Präkonzentrate bestehend aus CsA, einer hydrophilen Phase (Propylenglykol oder Partialether von niedermolekularen Mono- bzw. Polyoxyalkandiolen (Transcutol/Glycofurol), einer lipophilen Phase (mittelkettige Triglyceride) und einem Tensid (Cremophor RH 40). Die Systeme sind geeignet für die perorale Applikation und verbessern die Bioverfügbarkeit verglichen mit vorhandenen Systemen.

Die EP 760237 beschreibt O/W-Mikroemulsionen für wasserunlösliche pharmazeutisch aktive Substanzen wie CsA, die vollständig gelöst sind in den dispergierten Öltröpfchen. Die Systeme bestehen aus einem C₈-C₂₀ substituierten pflanzlichen Triglycerid, Lecithin sowie einem anderen Tensid und einer Propylenglykol enthaltenden hyrophilen Phase.

Die WO 97/22358 beinhaltet Mikroemulsions-Präkonzentrate mit CsA, wobei der Arzneistoff in einem System, bestehend aus hydrophoben (Tocopherolen bzw. - derivaten) und hydrophilen Komponenten (Propylencarbonat und Polyethylenglycol mit Molekulargewicht < 1000 sowie Tensid, gelöst ist.

In WO 94/08603, WO 94/08605 und WO 99/39700 sind Arzneimittelformulierungen beschrieben, die als möglichen Wirkstoff im kolloidalen System Cyclosporin und dessen Derivate aufführen.

Die bisher beschriebenen Systeme weisen jedoch einige wesentliche Nachteile auf. Zur Solubilisierung werden teilweise organische Lösungsmittel verwendet, die anschließend wieder aus der Formulierung rückstandsfrei entfernt werden müssen. Oftmals werden Tensid/Cotensid-Kombinationen zur Löslichkeitsverbesserung des Arzneistoffes in zu hohen Konzentrationen (mehr als 20% m/m) eingesetzt. Einige Publikationen erwähnen Systeme, die nicht ausschließlich aus hautverträglichen Inhaltsstoffen zusammengesetzt sind. Es werden einige Mikroemulsions-Präkonzentrate beschrieben, deren eigentliche Struktur sich erst nach Applikation in situ ausbilden soll. Desweiteren weisen die vorhandenen Systeme weitaus größere Teilchendurchmesser auf.

Aufgabe der vorliegenden Erfindung ist es deshalb, ein neues kolloidales Arzneistoffträgersystem vorzuschlagen, das im wesentlichen aus dermatologisch verträglichen Inhaltsstoffen besteht, wobei dieses Arzneistoffträgersystem gleichzeitig verhältnismäßig geringe Tensid/Cotensidgehalte aufweisen sollte.

Die Aufgabe wird durch die kennzeichnenden Merkmale des Patentanspruches 1 gelöst. In bezug auf die Verwendung wird die Erfindung durch die Merkmale der Ansprüche 14 bis 20 gelöst.

Erfindungsgemäß wird somit vorgeschlagen, daß die Arzneiformulierung in kolloidaler Form aus vier wesentlichen Bestandteilen besteht. Die erfindungsgemä-ße Arzneiformulierung enthält somit eine lipophile Phase, eine Mischung aus Tensid und Cotensid in einem bestimmten Massenverhältnis, eine hydrophile Phase sowie als Wirkstoff Ciclosporin in den in Anspruch 1 angegebenen Konzentrationen.

Der Vorteil der erfindungsgemäßen kolloidalen Arzneistoffträgersysteme ist insbesondere in der Zusammensetzung aus ausschließlich dermatologisch verträglichen Inhaltsstoffen, in verhältnismäßig'geringen Tensid/Cotensidgehalt sowie der geringen Teilchengröße der dispergierten Partikel zu sehen.

Aus stofflicher Sicht eignen sich für die lipophile Phase für die Arzneiformulierung nach der Erfindung insbesondere Öle, Wachse oder Fette. In bezug auf die lipophile Phase können für sich alle aus dem Stand der Technik bisher bekannten lipophilen Phasen eingesetzt werden. Besonders bevorzugt sind Triglyceride, Isopropylpmyristat, 2-Octyldodecanol, Isopropylpalmitat oder Ölsäure. Die lipophile Phase ist mit 1-10,Gew.-% in der Formulierung enthalten.

Wesentliches Element der Arzneiformulierung nach der Erfindung ist die Mischung aus Tensid und Cotensid, die in einer Menge von 1 bis 50 Gew.-%, bevorzugt von 20-30 Gew.-% eingesetzt wird. Aus stofflicher Sicht sind Tenside ausgewählt aus Polyoxyethylenglycerolfettsäureestern und Polyoxyethylensorbitanfettsäureestern bevorzugt. Beispiele für das Cotensid sind Poloxamere, Blockcopolymere aus Polyoxyethylen und Polyoxypropylen.

Das Mischungsverhältnis ist ein Masseverhältnis von 1,5 bis 2,5 für das Tensid und 2,5 bis 3,5 für das Cotensid. Die Erfinder konnten nachweisen, daß insbesondere die Einhaltung dieses Tensid/Cotensid-Mischungsverhältnisses für die Stabilität und die Anwendbarkeit der Arzneiformulierung wichtig ist.

Wenn die Tensid/Cotensid-Mischung aus Polyoxyethylenglycerolfettsäureestern und Poloxameren in einem Masseverhältnis von 2:3 besteht, konnten besonders gute Ergebnisse erzielt werden.

Die erfindungsgemäße Formulierung enthält weiterhin eine hydrophile Phase die, wie an und für sich aus dem Stand der Technik bekannt, aus Polyolen, Wasser. oder einem Polyol oder einer Polyol-Puffer-Mischung oder nur Puffer, bestehen kann. Die Konzentration dieses Bestandteiles beträgt 40-80 Gew.-%, bevorzugt 60-75 Gew.-%.

Eine weitere vorteilhafte Ausgestaltung der Erfindung sieht vor, daß bei der hydrophilen Phase eine Mischung aus Propylenglycol und Wasser im Verhältnis von 1:10 bis 10:1 eingesetzt wird. Ein besonders bevorzugtes Mischungsverhältnis ist 2:1.

Erfindungsgemäß enthält die Arzneiformulierung als Wirkstoff Ciclosporin und/oder ein Derivat hiervon in einer Konzentration von 0,1-20 Gew.-%. Besonders bevorzugt ist es, wenn die Arzneiformulierung Ciclosporin A und/oder seine Derivate enthält. Die bevorzugte Konzentration liegt hierbei im Bereich von 0,01-10 Gew.-%, besonders bevorzugt bei 0,5-5 Gew.-%.

Selbstverständlich ist es auch möglich, daß in der Arzneiformulierung neben Ciclosporin A und/oder seinen Derivaten ein weiterer Wirkstoff enthalten ist. Beispiel für derartige Wirkstoffe sind Corticosteroide, Antibiotika, Antimykotika und/oder Virustatika.

Wie an und für sich aus dem Stand der Technik bekannt, können der Arzneiformulierung nach der Erfindung auch die üblichen Additive wie Penetrationsenhancer zugesetzt werden. Wenn Penetrationsenhancer zugesetzt werden, sind Dimethylsulfoxid oder kurzkettige Alkohole in einer Konzentration von 5-10 Gew.-% bevorzugt.

Die erfindungsgemäße Arzneiformulierung liegt in kolloidaler Form vor. Bevorzugt ist es hierbei, wenn die disperse Phase Teilchendurchmesser in der Größenordnung von 5 bis 200 nm aufweist. Besonders bevorzugt liegen die Teilchendurchmesser im Größenbereich von 5 bis 100 nm.

Die erfindungsgemäße Arzneiformulierung ist besonders geeignet zur Prophylaxe entzündlicher Haut- und Schleimhauterkrankungen, zur Therapie und Prophylaxe der atopischen Dermatitis, zur Therapie und Prophylaxe der Psoriasis vulgaris.

Andere geeignete Verwendungen sind die Therapie und Prophylaxe von Kollagenosen, chronischen Wunden, Verbrennungen und/oder chronisch entzündlichen Haut- und Schleimhauterkrankungen sowie zur Therapie und Prophylaxe von chronisch entzündlichen Darmerkrankungen und zur Therapie und Prophylaxe von entzündlichen Erkrankungen des Auges und nach Transplantationen.

Nachfolgend wird die Erfindung anhand verschiedener Zusammensetzungen und Untersuchungsergebnisse näher beschrieben.

### Zusammensetzung und Herstellung der Vehikelsysteme

Es wurden drei kolloidale Arzneistoffträgersysteme entwickelt, deren Zusammensetzung aus Tabelle 1 ersichtlich ist.

**Tab. 1: Entwickelte kolloidale Arzneistoffträgersysteme**

| **IPP-System** | | **m[g]** | **% [w/w]** |
|---|---|---|---|
| Ciclosporin A 2,0% (w/w) | Ciclosporin A | 0,2 | 2,0 |
| Tagat^{®} O2/Synperonic^{®} PE/L 101 2:3 | Tagat^{®} O2 | 0,8 | 8,0 |
| 20,0% (w/w) | Synperonic^{®} PE/L 101 | 1,2 | 12,0 |
| Isopropylpalmitat (IPP) 5,0% (w/w) | Isopropylpalmitat | 0,5 | 5,0 |
| Propylenglykol/Wasser 2:1 73,0% (w/w) | Propylenglykol | 4,87 | 48,7 |
| | Wasser | ad | 24,3 |
| | | 10,0 | |
| **DMSO-System** | | | |
| Ciclosporin A 2,0% (w/w) | Ciclosporin A | 0,2 | 2,0 |
| Tagat^{®} 02/Synperonic^{®} PE/L 121 2:3 | Tagat^{®} O2 | 0,8 | 8,0 |
| 20,0% (w/w) | Synperonic^{®} PE/L 121 | 1,2 | 12,0 |
| Ölsäure 5,0% (w/w) | Ölsäure | 0,5 | 5,0 |
| Dimethylsulfoxid (DMSO) 5,0% (w/w) | Dimethylsulfoxid | 0,5 | 5,0 |
| Propylenglykol/Wasser 2:1 68,0% (w/w) | Propylenglykol | 4,53 | 45,3 |
| | Wasser | ad | 22,7 |
| | | 10,0 | |
| **ÖS-System** | | | |
| Ciclosporin A 1,5% (w/w) | Ciclosporin A | 0,15 | 1,5 |
| Tagat^{®}O2/Synperonic^{®} PE/L 121 2:3 | Tagat^{®}O2 | 0,8 | 8,0 |
| 20,0% (w/w) | Synperonic^{®} PE/L 121 | 1,2 | 12,0 |
| Ölsäure 5,0% (w/w) | Ölsäure | 0,5 | 5,0 |
| Propylenglykol/Wasser 2:1 73,5% (w/w) | Propylenglykol | 4,9 | 49,0 |
| | Wasser | ad | 24,5 |
| | | 10, 0 | |

Die Herstellung der Systeme erfolgte durch die konkrete Abfolge der folgenden Schritte:
- Einwiegen des Arzneistoffs
- Zugabe der Tensid/Cotensidmischung
- gut verreiben
- Zugabe der erforderlichen Menge IPP
- gut mischen
- Zugabe der hergestellten Mischung aus Propylenglykol und Wasser
- Rühren bis zum Klarwerden, evtl. kurze Behandlung des kolloidalen Systems mit Ultraschall

Als Emulgatoren wurden Polyoxyethylenglycerolmonooleat (Tagat^{®} O2) sowie Poloxamere (Synperonic^{®} PE/L 101 bzw. 121) ausgewählt. Für die Herstellung der Vehikelsysteme hat sich dabei eine Kombination aus beiden im Verhältnis 2:3 Massenanteile als besonders geeignet erwiesen.

Mischungen der beiden Tenside sowie von Propylenglykol/Wasser wurden im voraus hergestellt. Zunächst wurde der pulverisierte Arzneistoff mit der Tensidmischung sorgfältig angerieben und dann die lipophile Phase (Isopropylpalmitat oder Ölsäure) zugesetzt. Anschließend erfolgte die Zugabe der hydrophilen Phase (Propylenglykol/Wasser-Mischung) und Rühren bis zum Klarwerden. DMSO wurde zuletzt eingearbeitet. Falls erforderlich, wurden die Systeme für einige Minuten im Ultraschallbad belassen.

Als lipophile Komponenten wurden Isopropylpalmitat bzw. Ölsäure eingesetzt, die beide als Lösungsmittel für CsA fungieren. Weiterhin erfüllt die Ölsäure die Funktion eines Penetrationsenhancers, um die Permeation des CsA durch das Stratum corneum zu erleichtern. Dimethylsulfoxid wurde zugesetzt zur Verbesserung der Löslichkeit des CsA im Vehikel und wegen seiner penetrationsfördernden Eigenschaften. Als Tensid/Cotensid-Mischung wurden Polyoxyethylenglycerolmonooleat, das in 100%iger Konzentration reaktionslos von Humanhaut vertragen wird und gut schleimhautverträglich ist, sowie Poloxamere, die für die intravenöse Administration zugelassen sind, ausgewählt.

### Charakterisierung der Vehikelsysteme

Die Arzneistoffträgersysteme wurden u.a. mit Hilfe der dynamischen Laserlichtstreuung charakterisiert. Dieses Verfahren ist zur Größenbestimmung kolloidaler Partikel in flüssigen Medien geeignet. Für die arzneistofffreien Formulierungen konnten Teilchendurchmesser von circa 20 nm ermittelt werden.

### Analytik

Die Bestimmung des CsA erfolgte mit Hilfe einer HPLC-Methode (modifiziert nach Merck KgaA - Darmstadt). Die technischen Daten sind aus Tabelle 2 ersichtlich.

**Tab. 2: Analytische Daten**

| | |
|---|---|
| HPLC-Anlage | Merck-Hitachi |
| | L-4250 UV-VIS Detektor |
| | AS-4000A Intelligent Auto-sampler |
| | D-6000A Interface |
| | L-6200A Intelligent Pump |
| stationäre Phase | Lichrospher^{®} RP select B (Merck), 125x4 mm ID, 5 µm |
| mobile Phase | Acetonitril/Wasser 70/30 (VN) |
| Fluß | 1 ml/min, isokratisch |
| Temperatur | 60°C |
| Detektion | UV 210 nm |

### Liberationsuntersuchungen

Mit Hilfe eines Mehrschichtmembranmodellsystems wurde die in-vitro-Freisetzung des Arzneistoffs aus den oben genannten Formulierungen in Abhängigkeit von der Zeit untersucht.

Die einzelnen Zellen des Modells bestanden jeweils aus Grund- und Deckscheibe, zwischen denen die Membranschichten angeordnet wurden. Über eine Aussparung der Deckscheibe (4cm²) wurde eine definierte Menge an Formulierung (10-20mg) auf die Membranen appliziert.
Als Akzeptor dienten Dodecanol-Collodium-Membranen mit einem Gehalt von 2% Dodecanol, die mit Hilfe eines Filmziehgeräts hergestellt wurden. Über die Bestimmung der Sättigungslöslichkeit von CsA in Dodecanol konnte die Aufnahmekapazität des Akzeptors ermittelt werden. Dies ist wichtig, um sicherzustellen, daß das Erreichen der Sättigungslöslichkeit, des Arzneistoffs in den Membranen nicht der limitierende Faktor der Liberation ist. Durch die Verwendung von drei übereinander gelegten Membranen wurden Sink-Bedingungen im Akzeptor gewährleistet.

Während der Versuchsdauer (30, 100, 300 und 1000 Minuten) wurde das Modell bei 32±1°C temperiert. Nach Ablauf der Versuchszeit wurde die überschüssige Formulierung vorsichtig entfernt, die Membranen getrennt, mit einem Ethanol-Wasser-Gemisch (80/20; V/V) extrahiert und einer Gehaltsbestimmung mittels HPLC unterzogen. Es wurde pro Versuchszeit eine Fünffachbestimmung durchgeführt.

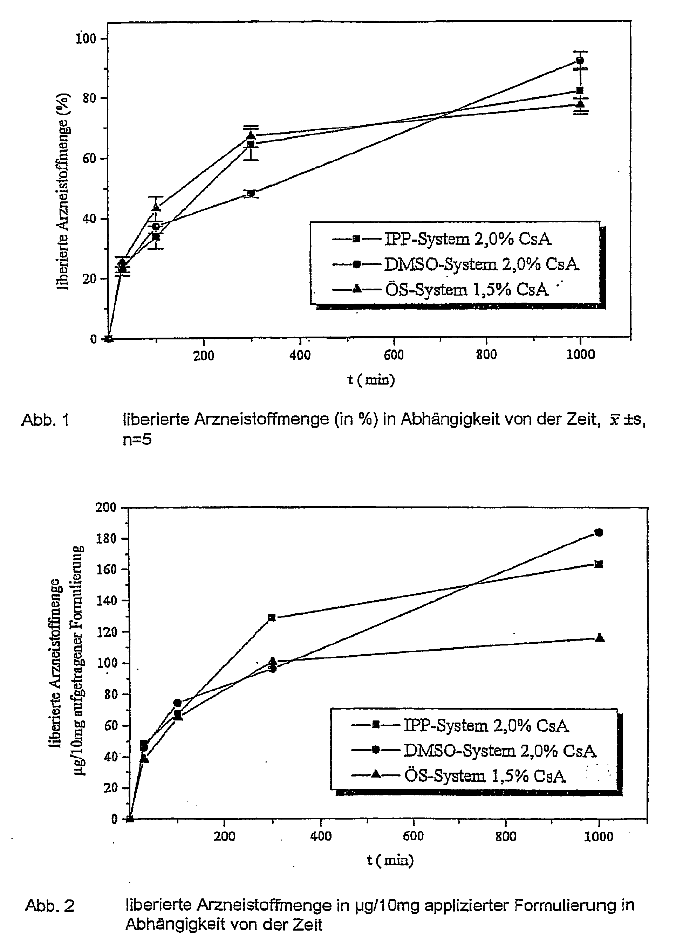

Aus Abbildung 1 ist erkennbar, daß alle drei Formulierungen bereits nach 30 Minuten ~25% des enthaltenen CsA freisetzen. Bei längeren Versuchszeiten erhöht sich die liberierte Arzneistoffmenge. In Abbildung 2 sind zum besseren Vergleich der unterschiedlich konzentrierten Vehikel die liberierten Arzneistoffmengen pro 10 mg applizierter Formulierung dargestellt.

Diese Untersuchungen wurden durchgeführt, um zu gewährleisten, daß eine ausreichende Freisetzung von CsA aus den Vehikeln erfolgt und damit die Voraussetzung für eine Penetration in die menschliche Haut erfüllt ist.

### Penetrationsuntersuchungen

Es wurde Humanhaut der Mamma verwendet, die durch Mammareduktionsplastiken gewonnen wurde. Die zugeschnittenen Hautstücke wurden bei -3°C gelagert. Nach dem Auftauen wurde die an der Oberfläche haftende Flüssigkeit mit einem Watteträger entfernt und die definierte Fläche von 3,14 cm² ausgestanzt. Auf die Oberfläche wurden etwa 6mg des radioaktiv markierten Testpräparates je cm² so aufgetragen, daß ein möglichst gleichmäßiger Film auf der Hautoberfläche entstand. Anschließend wurde das Hautstück auf einer Gaze liegend in die auf 32°C temperierte Franz'sche Diffusionszelle gespannt. Selbige wurde vor Versuchsbeginn im gefüllten Zustand einer 30minütigen Equilibrierungsphase unterzogen. An die Hautunterseite bzw. die Gaze grenzte direkt das Akzeptormedium, das zur Verringerung der Diffusionsschichtdicke stetig gerührt wurde. Um physiologische Bedingungen zu simulieren, wurde isotone NaCl-Lösung als Akzeptorflüssigkeit verwendet. Die Untersuchungen wurden jeweils an 3 verschiedenen Operationspräparaten als Dreifachbestimmungen durchgeführt. Nach Ablauf der Einwirkungsdauer wurden die Hautstücke herausgenommen und mit Hilfe von Stecknadeln auf einer mit Aluminiumfolie ummantelten Styroporbox fixiert. Anschließend wurde das Testpräparat mit einem Mulltupfer abgewischt.

Das Entfernen des Stratum corneum erfolgte durch eine Schablone, die eine kreisrunde Aussparung (d=16mm) enthielt. Durch diese Aussparung wurden 20 Tesafilmabrisse (Tesa Film^{®} 4 204, 33 m x 19 mm: Fa. Beiersdorf AG, Hamburg) von einer 2,0106 cm² großen Hautoberfläche entfernt. Jeweils zwei nacheinander gewonnene Abrisse wurden zusammen vermessen.

Aus dem übrigen Hautstück wurden mittels einer Kromayer-Stanze (Durchmesser 6 mm; Stiefel Laboratorium GmbH, Offenbach) etwa aus der Mitte des Hautareals 3 Zylinder mit einer Gesamtoberfläche von 0,848 cm² ausgestanzt. Mit einem Gefriermikrotom wurden die so gewonnenen Gewebszylinder nacheinander auf -40°C tiefgefroren und horizontal zur Hautoberfläche aufgeschnitten. Dabei wurden 10x20 µm-Schnitte zur Entfernung der vitalen Epidermisanteile und 15x80 µm-Schnitte zur Aufarbeitung des Koriums entnommen. Danach blieben ein Koriumrest (Stumpf) und das Akzeptormedium übrig.

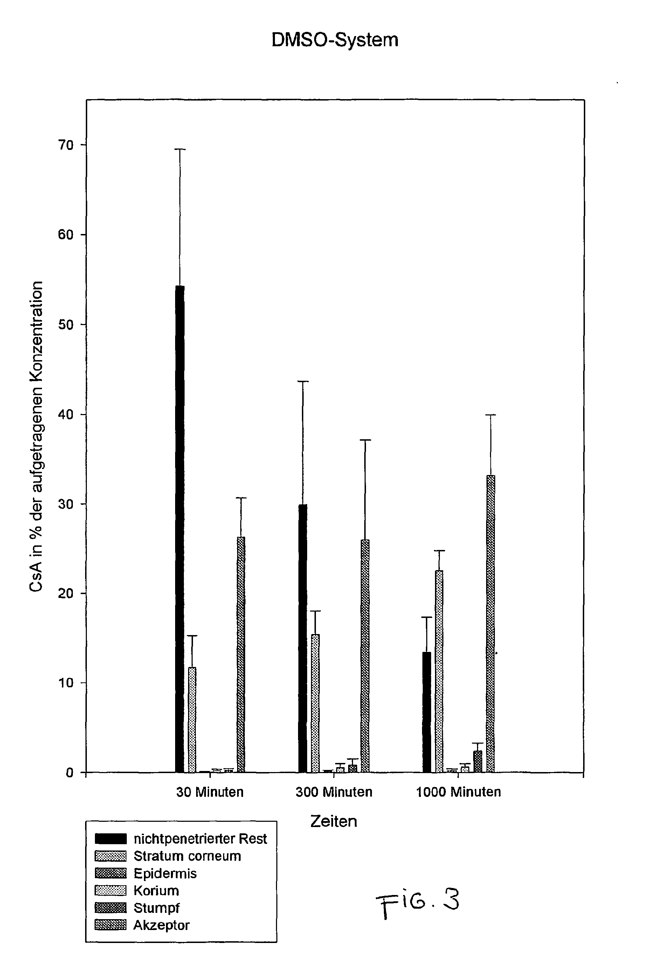

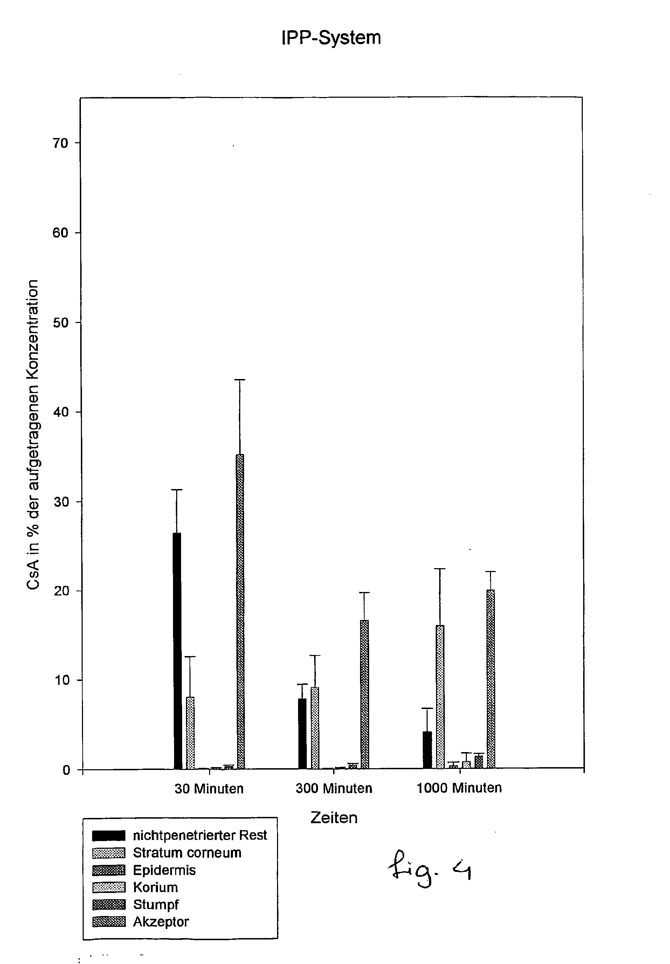

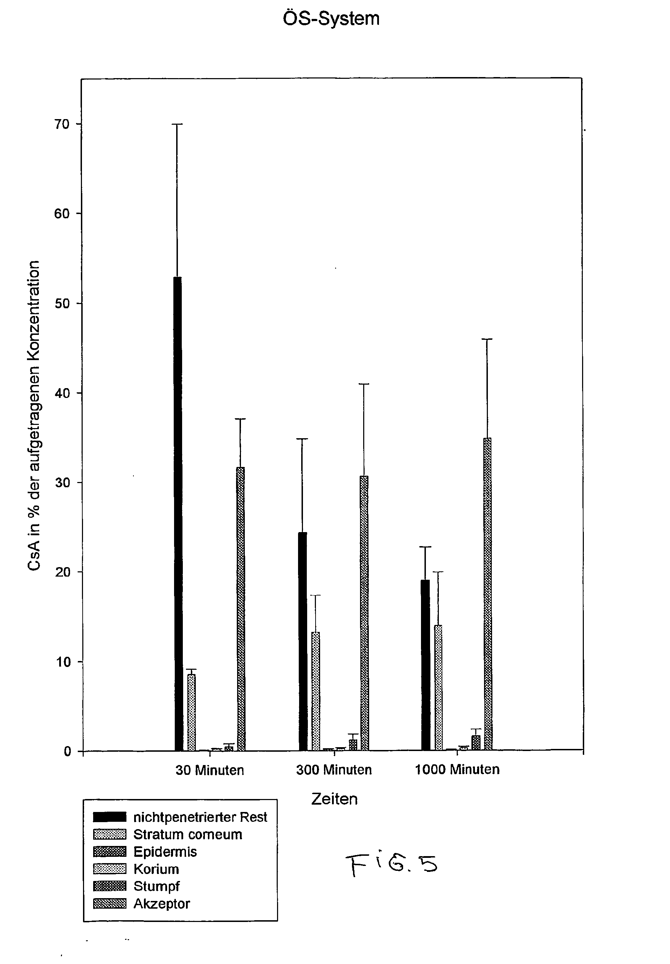

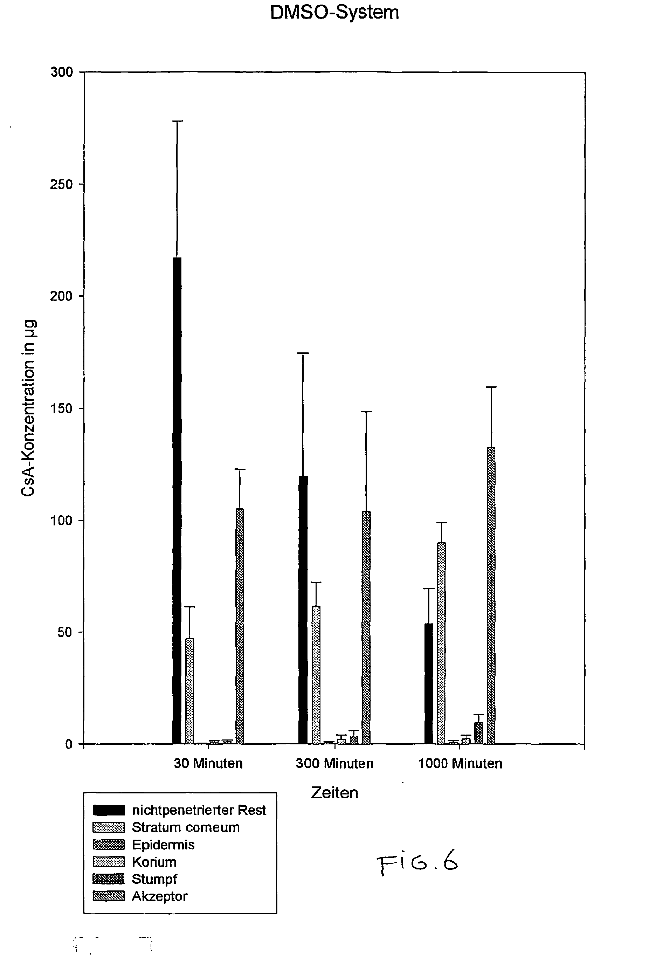

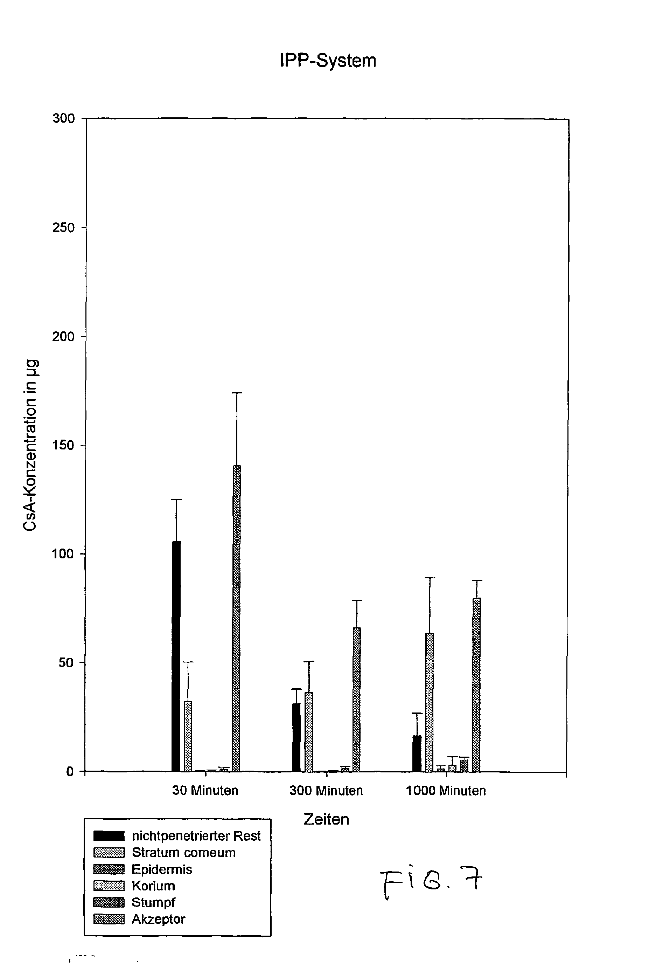

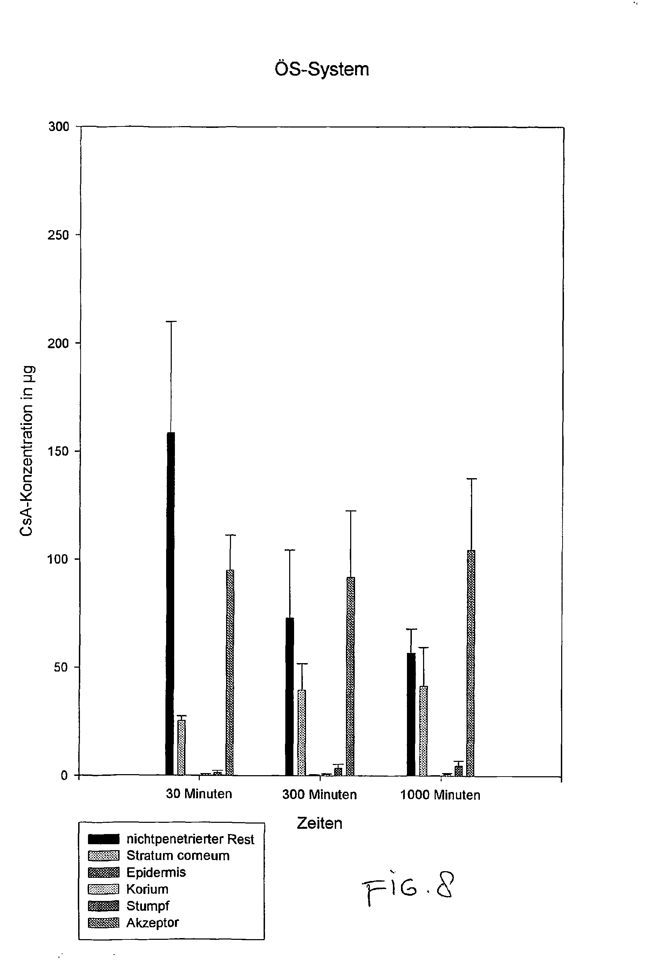

Aus den Abbildungen 3-5 ist ersichtlich, daß bei allen drei Formulierungen bereits nach 30 Minuten >25% (IPP 35%, ÖS 32%, DMSO 27%) des enthaltenen CsA bis in den Akzeptor penetriert sind. Bei längeren Versuchszeiten erhöht sich die penetrierte Arzneistoffmenge geringfügig. In den Abbildungen 6-8 sind zum besseren Vergleich der unterschiedlich konzentrierten Vehikel die penetrierten Arzneistoffmengen in µg dargestellt.

Die bisher publizierten galenischen Daten von ciclosporinhaltigen Präparationen für die topische Anwendung zeigen eine unzureichende Liberation und/oder Penetration der Wirksubstanz. Dabei sind hydrophile und lipophile Standardsysteme bzw. liposomale Zubereitungen untersucht worden. Vielfach finden sich auch in den Publikationen keine exakten Hinweise auf die Inhaltsstoffe der verwendeten Präparationen, die Art der Herstellung oder die galenischen Daten. Für den ausbleibenden klinischen Effekt werden zwei Gründe angegeben. Erstens geht man wegen der starken Lipophilie von CsA von einer zu geringen Liberation und/oder Penetration des Wirkstoffes in die korialen Schichten der Haut aus. Zweitens wird in der hohen molaren Masse ein deutlich penetrationsinhibierender Faktor gesehen. Die vorliegenden Ergebnisse zeigen erstmals Liberations- und Penetrationsraten von ca. 25-30% der applizierten Wirkstoffkonzentration in den ersten 30 bis 100 Minuten an gesunder, also barriereintakter Haut ex vivo. Grundsätzlich ist an läsionaler Haut (z.B. psoriatischer Plaque) im Vergleich zu gesunder Haut von günstigeren Penetrationsbedingungen auszugehen.

Die topische Applikation von CsA ergibt im Vergleich zur systemischen Therapie deutliche Vorteile. Bei einer topischen Applikation ist auch bei großflächiger Behandlung mit minimalen und nicht klinisch relevanten systemischen Nebenwirkungen zu rechnen. Die bekannten unerwünschten Arzneistoffwirkungen, insbesondere Nierenfunktionsstörung und arterielle Hypertonie, sind nicht oder in einem geringeren Maße zu erwarten. Eine Verwendung kann sogar für Patienten in Frage kommen, die bereits durch eine systemische Therapie Nebenwirkungen entwickelt haben Und deshalb nicht mehr mit CsA behandelt werden. Darüber hinaus ergibt sich durch die topische Applikation ein erweitertes Wirkungsspektrum. Bei einer Vielzahl von entzündlichen Dermatosen steht die Bildung und Wirkung von Stickstoffmonoxid (NO), welches aus L-Arginin durch das Enzym NO-Synthase gebildet wird, im Zentrum der Pathogenese. Jüngste Forschungsergebnisse zeigen, daß die Vermittlung und Unterhaltung der Entzündung bei der Psoriasis wesentlich durch NO vermittelt wird. CsA ist bekanntlich ein suffizienter Blocker der NOS, die durch die Isoenzyme eNOS und iNOS auch in dermalen mikrovaskulären Endothelzellen präsent ist und durch eine hohe Gewebekonzentration an CsA nach topischer Applikation stärker inhibiert wird als nach systemischer Applikation. Zudem hat CsA auch einen proliferationsinhibierenden Effekt auf Keratinozyten, der bei topischer Applikation auf psoriatischer Haut synergistisch die Wirkung beeinflußt. Günstig wäre auch eine Kosteneindämmung pro Fall durch die geringere benötigte Menge an CsA.

Nachteile der topischen Applikation und der damit verbundenen erniedrigten Systemkonzentration werden vor allem in der ausbleibenden Wirkung auf die Lymphknoten-gesehen, in denen wesentliche Aktivierungsvorgänge auf T-zellulärer Ebene ablaufen. Darüber hinaus wird ein therapeutischer Effekt bei Psoriasis arthropathica nicht zu erwarten sein.

Die vorliegenden Untersuchungen weisen erstmals nach, daß es gelungen ist, ein galenisches System zu entwickeln, welches die Voraussetzungen für eine Penetration von CsA in ausreichender Konzentration bis in die oberen Koriumschichten erfüllt. Es liegen stabile Vehikelsysteme vor, in die ausreichend CsA eingearbeitet werden kann. Die Beladung des Systems ist absichtlich sehr hoch gewählt worden, um die galenischen Eigenschaften der Systeme auszureizen und günstige Voraussetzungen für eine klinische Applikation zu schaffen. Geringere CsA-Konzentrationen sind bei ausreichender klinischer Wirksamkeit problemlos zu realisieren. Die Liberationsuntersuchungen weisen nach, daß in relevanter Zeit Arzneistoffmengen zwischen 25 und 40% freigesetzt werden und für die Penetration in die Haut zur Verfügung stehen.

Von entscheidender Bedeutung für die präklinische Entwicklung werden die Penetrationsuntersuchungen unter ex vivo-Bedingungen bewertet. Sie verdeutlichen, daß nach relevanter Applikationsdauer (30-100 min) eine Penetration von ca. 25-30% der Wirkstoffkonzentration in bzw. durch die korialen Schichten erfolgt und somit am gewünschten Wirkort zur Verfügung stehen.

Die Inhaltsstoffe der Präparationen sind nach dermatologischen Gesichtspunkten ausgewählt worden. Es sind keine hochpotent sensibilisierenden Substanzen enthalten, und Selbstversuche haben eine gute Verträglichkeit gezeigt, die eine irritative oder toxische Wirkung auch unter Barrierefunktionsstörungen bei verschiedenen Dermatosen sehr unwahrscheinlich erscheinen lassen. Als Hauptindikationsgebiete ergeben sich die Psoriasis vulgaris vom chronisch stationären Typ bzw. die atopische Dermatitis. Darüber hinaus ergeben sich in Anlehnung an die systemische Therapie eine Vielzahl von möglichen Indikationen. Insbesondere wird der Einsatz bei Kollagenosen, Verbrennungen, Haut- oder Schleimhauttransplantationen und chronischen Wunden für möglich gehalten.

Die Anwendung der Systeme ist nicht nur für die äußere Haut vorgesehen. Grundsätzlich ergibt sich auch die Möglichkeit einer Applikation am Auge nach Hornhauttransplantation oder zur Therapie des vernarbenden Schleimhautpemphigoids bzw. eine Applikation im Mundschleimhautbereich bei Lichen ruber mucosae bzw. als Klistier bei chronisch entzündlichen Darmerkrankungen (z.B. M. Crohn, Colitis ulcerosa) ggf. auch durch intraluminale Aufschäumung.

## Patentansprüche

1. Arzneiformulierung in kolloidaler Form zur topischen Anwendung für die Therapie und Prophylaxe krankhafter Veränderungen der Haut und/oder Hautanhangsgebilde und/oder Schleimhäute, einschließlich Schleimhäute des Verdauungstraktes, Urogenitaltraktes sowie Bronchialsystems und/oder Konjunktiven, enthaltend
a) eine lipophile Phase in einer Menge von 1-10 Gew.-%,
b) eine Mischung aus Tensid und Cotensid in einer Menge von 1-50 Gew.-%, wobei die Tensid-Cotensid-Mischung in einem Massenverhältnis von 1,5 bis 2,5 für das Tensid und 2,5 bis 3,5 für das Cotensid eingesetzt ist,
c) eine hydrophile Phase in einer Menge von 40-80 Gew.-% und
d) als Wirkstoff Ciclosporin und/oder Derivate hiervon in einer Konzentration von 0,1-20 Gew.-%.

2. Arzneiformulierung nach Anspruch 1, **dadurch gekennzeichnet, dass** die lipophile Phase ausgewählt ist aus pharmazeutischen Ölen, Wachsen oder Fetten.

3. Arzneiformulierung nach Anspruch 2, **dadurch gekennzeichnet, dass** die lipophile Phase ausgewählt ist aus Triglyceriden, Isopropylmyristat, 2-Octyldodecanol, Isopropylpalmitat oder Ölsäure.

4. Arzneiformulierung nach mindestens einem der Ansprüche 1 bis 3,
**dadurch gekennzeichnet, dass** das Tensid ausgewählt ist aus Polyoxyethylenglycerolfettsäureestern und Polyoxyethylensorbitanfettsäureestern.

5. Arzneiformulierung nach mindestens einem der Ansprüche 1 bis 4, **dadurch gekennzeichnet, dass** das Cotensid ausgewählt ist aus Poloxameren, Blockcopolymere aus Polyoxyethylen und Polyoxypropylen.

6. Arzneiformulierung nach mindestens einem der Ansprüche 1 bis 5, **dadurch gekennzeichnet, dass** die Tensid-Cotensid-Mischung aus Polyoxyethylenglycerolfettsäureester und Poloxameren in einem Massenverhältnis von 2:3 besteht.

7. Arzneiformulierung nach mindestens einem der Ansprüche 1 bis 6, **dadurch gekennzeichnet, dass** die hydrophile Phase ausgewählt ist aus Polyolen, Wasser oder Polyol-Puffer-Mischungen.

8. Arzneiformulierung nach Anspruch 7, **dadurch gekennzeichnet, dass** die hydrophile Phase eine Mischung aus Propylenglycol und Wasser im Verhältnis von 1:10 bis 10:1, bevorzugt 2:1 ist.

9. Arzneiformulierung nach mindestens einem der Ansprüche 1 bis 8, **dadurch gekennzeichnet, dass** Ciclosporin A und/oder seine Derivate in einer Konzentration von 0,01-10 Gew.-%, bevorzugt 0,5-5 Gew.-% enthalten sind.

10. Arzneiformulierung nach mindestens einem der Ansprüche 1 bis 9, **dadurch gekennzeichnet, dass** neben Ciclosporin A und/oder seinen Derivaten mindestens ein weiterer Wirkstoff, z. B. Corticosteroid enthalten ist.

11. Arzneiformulierung nach mindestens einem der Ansprüche 1 bis 10, **dadurch gekennzeichnet, dass** zusätzlich Penetrationsenhancer, wie z. B. Dimethylsulfoxid oder kurzkettige Alkohole in einer Konzentration von 5-10 Gew.-% enthalten sind.

12. Arzneiformulierung nach mindestens einem der Ansprüche 1 bis 11, **dadurch gekennzeichnet, dass** die disperse Phase Teilchendurchmesser in der Größenordnung von 5 bis 200 nm aufweist.

13. Verwendung der Arzneiformulierung nach einem oder mehreren der Ansprüche 1 bis 12, zur Herstellung eines Arzneimittels zur Therapie und Prophylaxe entzündlicher Haut- und Schleimhauterkrankungen.

14. Verwendung der Arzneiformulierung nach Anspruch 13, zur Therapie und Prophylaxe der atopischen Dermatitis.

15. Verwendung der Arzneiformulierung nach Anspruch 13, zur Therapie und Prophylaxe der Psoriasis vulgaris.

16. Verwendung der Arzneiformulierung nach Anspruch 13, zur Therapie und Prophylaxe von Kollagenosen, chronischen Wunden, Verbrennungen und/oder chronisch entzündlichen Haut- und Schleimhauterkrankungen.

17. Verwendung der Arzneiformulierung nach Anspruch 13, zur Therapie und Prophylaxe von chronisch entzündlichen Darmerkrankungen.

18. Verwendung der Arzneiformulierung nach Anspruch 13, zur Therapie und Prophylaxe von entzündlichen Erkrankungen des Auges.

19. Verwendung der Arzneiformulierung nach Anspruch 13, zur Therapie und Prophylaxe von Abstoßungsreaktionen nach Transplantationen.

## Claims

1. Pharmaceutical formulation in colloidal form for topical application for the therapy and prophylaxis of pathological changes of the skin and/or integumentary structures of the skin and/or mucous membranes, including mucous membranes of the digestive tract, uro-genital tract and bronchial system and/or conjunctiva, containing
a) a lipophilic phase in a quantity of 1 - 10% by weight,
b) a mixture of surfactant and co-surfactant in a quantity of 1 - 50% by weight, the surfactant/ co-surfactant mixture being used in a mass ratio of 1.5 to 2.5 for the surfactant and 2.5 to 3.5 for the co-surfactant,
c) a hydrophilic phase in a quantity of 40 - 80% by weight and
d) as active ingredient, cyclosporin and/or derivatives thereof in a concentration of 0.1 - 20% by weight.

2. Pharmaceutical formulation according to claim 1, **characterised in that** the lipophilic phase is selected from pharmaceutical oils, waxes or fats.

3. Pharmaceutical formulation according to claim 2, **characterised in that** the lipophilic phase is selected from triglycerides, isopropyl myristate, 2-octyldodecanol, isopropyl palmitate or oleic acid.

4. Pharmaceutical formulation according to at least one of the claims 1 to 3,
**characterised in that**
the surfactant is selected from polyoxyethylene glycerol fatty acid esters and polyoxyethylene sorbitan fatty acid esters.

5. Pharmaceutical formulation according to at least one of the claims 1 to 4, **characterised in that** the co-surfactant is selected from poloxamers, block copolymers of polyoxyethylene and polyoxypropylene.

6. Pharmaceutical formulation according to at least one of the claims 1 to 5, **characterised in that** the surfactant/ co-surfactant mixture comprises polyoxyethylene glycerol fatty acid ester and poloxamers in a material ratio of 2 : 3.

7. Pharmaceutical formulation according to at least one of the claims 1 to 6, **characterised in that** the hydrophilic phase is selected from polyols, water or polyol-buffer mixtures.

8. Pharmaceutical formulation according to claim 7, **characterised in that** the hydrophilic phase is a mixture of propylene glycol and water in a ratio of 1 : 10 to 10 : 1, preferably 2 : 1.

9. Pharmaceutical formulation according to at least one of the claims 1 to 8, **characterised in that** cyclosporin A and/or derivatives thereof are contained in a concentration of 0.01 - 10% by weight, preferably 0.5 - 5% by weight.

10. Pharmaceutical formulation according to at least one of the claims 1 to 9, **characterised in that** at least one further active ingredient, e.g. corticosteroid, is contained in addition to cyclosporin A and/or derivatives thereof.

11. Pharmaceutical formulation according to at least one of the claims 1 to 10, **characterised in that** in addition penetration enhancers, such as e.g. dimethyl sulphoxide or short-chain alcohols, are contained in a concentration of 5 - 10% by weight.

12. Pharmaceutical formulation according to at least one of the claims 1 to 11, **characterised in that** the disperse phase has particle diameters of the order of magnitude of 5 to 200 nm.

13. Use of the pharmaceutical formulation according to one or more of the claims 1 to 12, in order to produce a pharmaceutical for the therapy and prophylaxis of inflammatory skin and mucous membrane diseases.

14. Use of the pharmaceutical formulation according to claim 13, for the therapy and prophylaxis of atopical dermatitis.

15. Use of the pharmaceutical formulation according to claim 13, for the therapy and prophylaxis of psoriasis vulgaris.

16. Use of the pharmaceutical formulation according to claim 13, for the therapy and prophylaxis of collagenoses, chronic wounds, burns and/or chronically inflammatory skin and mucous membrane diseases.

17. Use of the pharmaceutical formulation according to claim 13, for the therapy and prophylaxis of chronically inflammatory bowel diseases.

18. Use of the pharmaceutical formulation according to claim 13, for the therapy and prophylaxis of inflammatory diseases of the eye.

19. Use of the pharmaceutical formulation according to claim 13, for the therapy and prophylaxis of rejection reactions after transplants.

## Revendications

1. Formulation pharmaceutique sous forme colloïdale destinée à une application topique pour la thérapie et la prophylaxie des changements pathologiques de la peau et/ou des annexes de la peau et/ou des muqueuses, y compris les muqueuses de l'appareil digestif, de l'appareil uro-génital et du système bronchique et/ou des tissus conjonctifs, contenant
a) une phase lipophile en une quantité de 1 à 10 % en poids,
b) un mélange de tensioactif et de cotensioactif en une quantité de 1 à 50 % en poids, le mélange tensioactif/cotensioactif étant utilisé en un rapport massique de 1,5 à 2,5 pour le tensioactif et de 2,5 à 3,5 pour le cotensioactif,
c) une phase hydrophile en une quantité de 40 à 80 % en poids et
d) en tant que principe actif, de la cyclosporine et/ou des dérivés de celle-ci en une concentration de 0,1 à 20 % en poids.

2. Formulation pharmaceutique selon la revendication 1, **caractérisée en ce que** la phase lipophile est choisie parmi les huiles, les cires ou les graisses pharmaceutiques.

3. Formulation pharmaceutique selon la revendication 2, **caractérisée en ce que** la phase lipophile est choisie parmi les triglycérides, le myristate d'isopropyle, le 2-octyldodécanol, le palmitate d'isopropyle ou l'acide oléique.

4. Formulation pharmaceutique selon au moins l'une quelconque des revendications 1 à 3, **caractérisée en ce que** le tensioactif est choisi parmi les esters d'acide gras de polyoxyéthylène glycérol et les esters d'acide gras de polyoxyéthylène sorbitane.

5. Formulation pharmaceutique selon au moins l'une quelconque des revendications 1 à 4, **caractérisée en ce que** le tensioactif est choisi parmi les poloxamères, les copolymères blocs constitués de polyoxyéthylène et de polyoxypropylène.

6. Formulation pharmaceutique selon au moins l'une quelconque des revendications 1 à 5, **caractérisée en ce que** le mélange tensioactif/cotensioactif est constitué d'esters d'acide gras de polyoxyéthylène glycérol et de poloxamères selon un rapport massique de 2/3.

7. Formulation pharmaceutique selon au moins l'une quelconque des revendications 1 à 6, **caractérisée en ce que** la phase hydrophile est choisie parmi les polyols, l'eau et les mélanges solution tampon - polyol.

8. Formulation pharmaceutique selon la revendication 7, **caractérisée en ce que** la phase hydrophile est un mélange de polypropylène glycol et d'eau selon un rapport de 1/10 à 10/1, et qui est de préférence égal à 2/1.

9. Formulation pharmaceutique selon au moins l'une quelconque des revendications 1 à 8, **caractérisée en ce qu'**elle contient de la cyclosporine A et/ou ses dérivés en une concentration de 0,01 à 10 % en poids, de préférence de 0,5 à 5 % en poids.

10. Formulation pharmaceutique selon au moins l'une quelconque des revendications 1 à 9, **caractérisée en ce que**, outre la cyclosporine A et/ou ses dérivés, elle contient au moins un autre principe actif, par exemple un corticostéroïde.

11. Formulation pharmaceutique selon au moins l'une quelconque des revendications 1 à 10, **caractérisée en ce qu'**elle contient en outre un agent permettant d'accroître la pénétration, tel que par exemple le diméthylsulfoxyde ou des alcools à chaîne courte en une concentration de 5 à 10 % en poids.

12. Formulation pharmaceutique selon au moins l'une quelconque des revendications 1 à 11, **caractérisée en ce que** la phase dispersée présente un diamètre de particule de l'ordre de grandeur de 5 à 200 nm.

13. Utilisation de la formulation pharmaceutique selon une ou plusieurs des revendications 1 à 12, pour la préparation d'un médicament à des fins de thérapie et de prophylaxie d'affections inflammatoires de la peau et des muqueuses.

14. Utilisation de la formulation pharmaceutique selon la revendication 13, à des fins de thérapie et de prophylaxie de la dermatite atopique.

15. Utilisation de la formulation pharmaceutique selon la revendication 13, à des fins de thérapie et de prophylaxie du psoriasis vulgaris.

16. Utilisation de la formulation pharmaceutique selon la revendication 13, à des fins de thérapie et de prophylaxie de collagénoses, de brûlures, de plaies chroniques et/ou d'affections inflammatoires chroniques de la peau et des muqueuses.

17. Utilisation de la formulation pharmaceutique selon la revendication 13, à des fins de thérapie et de prophylaxie d'affections intestinales inflammatoires chroniques.

18. Utilisation de la formulation pharmaceutique selon la revendication 13, à des fins de thérapie et de prophylaxie d'affections inflammatoires de l'oeil.

19. Utilisation de la formulation pharmaceutique selon la revendication 13, à des fins de thérapie et de prophylaxie de réactions de rejet suite à des transplantations.
